# EUROPEAN PATENT APPLICATION

(11) **EP 4 079 289 A1**
(43) Date of publication of application: **26.10.2022**
(21) Application number: 21170004.2
(22) Date of filing: 22.04.2021
(51) Int. Cl.: A61K 9/00, A61K 9/08, A61K 47/26, A61K 47/36, A61P 11/00, A61P 31/12

(54) **ANTI-VIRAL COMPOSITION FOR ADMINISTRATION INTO NOSE, MOUTH OR THROAT**

(71) Applicant: Dutch Renewable Energy B.V., 1398 CP Muiden (NL)
(72) Inventor: PELLIKAAN, Hubert Clemens, 3572CA Utrecht (NL)
(74) Representative: Nederlandsch Octrooibureau

(57) **Abstract**

The invention provides an aqueous liquid composition for use in the prevention or treatment of a viral respiratory infection, said use comprising introducing the liquid aqueous composition into nose, mouth or throat of a human subject, said aqueous composition comprising:
• 0.01-5% by weight of carrageenan selected from lambda-carrageenan, iota-carrageenan, kappa-carrageenan and combinations thereof;
• 0.1-15% by weight of xylitol; and
• 0.01-5% by weight of anionic polysaccharide selected from pectin, alginate, gellan gum, xanthan gum and combinations thereof;
• 80-99% by weight water.

The invention also provides a metered-dose pump spray device comprising:
• a container holding the anti-viral composition of the present invention,
• a nozzle, and
• a manual pump connecting the nozzle and the container, said pump being configured to deliver one spray-unit upon actuation by manual force.

## Description

### TECHNICAL FIELD OF THE INVENTION

The present invention relates to an aqueous liquid composition for use in the prevention or treatment of a viral respiratory infection, said use comprising introducing the liquid aqueous composition into nose, mouth or throat of a human subject, said aqueous composition comprising (i) carrageenan selected from lambda-carrageenan, iota-carrageenan, kappa-carrageenan and combinations thereof; (ii) xylitol; and (iii) anionic polysaccharide selected from pectin, alginate, gellan gum, xanthan gum and combinations thereof.

The invention further relates to a method of preparing such an anti-viral aqueous liquid composition and to a metered-dose pump spray device comprising the anti-viral aqueous liquid composition.

### BACKGROUND OF THE INVENTION

Viral infections are among the most common afflictions of man. It has been estimated that children experience two to seven respiratory infections each year; adults are afflicted with one to three such episodes.

Viruses cause familiar infectious diseases such as the common cold, flu and warts. They also cause severe illnesses such as HIV/AIDS, Ebola, influenza and COVID-19.

Viral infections occur due to infection with a virus. Viruses contain a small piece of genetic code, and a coat of protein and lipid (fat) molecules protects them. Viruses invade a host and attach themselves to a cell. As they enter the cell, they release their genetic material. This material forces the cell to replicate the virus, and the virus multiplies.

Viruses spread in many ways. One transmission pathway is through disease-bearing organisms known as vectors: for example, viruses are often transmitted from plant to plant by insects that feed on plant sap, such as aphids; and viruses in animals can be carried by blood-sucking insects. Influenza viruses are spread by coughing and sneezing. Norovirus and rotavirus, common causes of viral gastroenteritis, are transmitted by the faecal-oral route, passed by hand-to-mouth contact or in food or water. HIV is one of several viruses transmitted through sexual contact and by exposure to infected blood.

Viral respiratory infection (VRI) is a general term for lung and airway infections. VRIs spread through contact with mucus from the mouth, throat or nose. Good hygiene practices, including handwashing, and covering the mouth and nose when coughing, can help prevent the spread of VRIs.

The spread of VRIs can also be prevented by administering anti-viral compositions to the mucus of nose, mouth or throat.

US 2012/0237572 describes a method of prophylactic or therapeutic treatment of a symptom, condition or disease caused by or associated with an infection by a respiratory virus selected from the group consisting of paramyxovirus, human influenza A virus, and adenovirus of subtype B, comprising administering to a subject a pharmaceutical composition comprising iota-carrageenan as an anti-viral active ingredient in an anti-viral effective amount.

Ferrer et al. (A Nasal Spray Solution of Grapefruit Seed Extract plus Xylitol Displays Virucidal Activity Against SARS-Cov-2 In Vitro, posted on November 25, 2020 on BioRxiv, the preprint server for biology) describe a nasal spray solution of grapefruit seed extract plus xylitol that displays *in vitro* anti-viral activity against SARS-Cov-2.

Moakes et al. (Formulation of a composite nasal spray enabling enhanced surface coverage and prophylaxis of SARS-COV-2, posted on November 18, 2020 on BioRxiv, the preprint server for biology) describe anti-viral nasal spray formulations containing gellan gum and lambda-carrageenan.

### SUMMARY OF THE INVENTION

The inventors have discovered that viral respiratory infections can be prevented effectively by administering at regular interval to the mucosa of nose, mouth or throat a liquid aqueous composition comprising:
- 0.01-5% by weight of carrageenan selected from lambda-carrageenan, iota-carrageenan, kappa-carrageenan and combinations thereof;
- 0.1-15% by weight of xylitol; and
- 0.01-5% by weight of anionic polysaccharide selected from pectin, alginate, gellan gum, xanthan gum and combinations thereof;
- 80-99 % by weight water.

The anionic polysaccharide in the anti-viral composition is capable of forming a gel when it comes into contact with dissolved calcium ions that are naturally present in the mucus. Thus, after introducing the anti-viral composition into nose, mouth or throat, a layer of gelled anti-viral composition is formed onto the surface of the mucus. The combination of carrageenan and xylitol that is contained in this gelled layer synergistically reduces the infectivity and transmissibility of viruses without adversely affecting the protective barrier properties of the mucus.

Another aspect of the invention relates to a method of preparing the anti-viral composition of the present invention, said method comprising:
- combining a source of carrageenan, xylitol, a source of anionic polysaccharide and aqueous liquid to produce an aqueous liquid mixture; and
- sterilizing the aqueous liquid mixture;
wherein the source of carrageen contains at least 50% by weight of dry mater of carrageenan selected from lambda-carrageenan, iota-carrageenan, kappa-carrageenan and combinations thereof; wherein the source of anionic polysaccharide contains at least 50% by weight of dry matter of anionic polysaccharide selected from pectin, alginate, gellan gum, xanthan gum and combinations thereof; and wherein the aqueous liquid contains at least 90 wt.% water.

The invention also provides a metered-dose pump spray device comprising:
- a container holding the anti-viral composition of the present invention,
- a nozzle, and
- a manual pump connecting the nozzle and the container, said pump being configured to deliver one spray-unit upon actuation by manual force.

### DETAILED DESCRIPTION OF THE INVENTION

A first aspect of the invention relates to an aqueous liquid composition for use in the prevention or treatment of a viral respiratory infection, said use comprising introducing the liquid aqueous composition into nose, mouth or throat of a human subject, said aqueous composition comprising:
- 0.01-5% by weight of carrageenan selected from lambda-carrageenan, iota-carrageenan, kappa-carrageenan and combinations thereof;
- 0.1-15% by weight of xylitol; and
- 0.01-5% by weight of anionic polysaccharide selected from pectin, alginate, gellan gum, xanthan gum and combinations thereof;
- 80-99% by weight water.

The term "carrageenan" as used herein refers to a family of natural linear sulfated polysaccharides that are extracted from seaweeds. The prevalent polysaccharides in carrageenan are designated as kappa-, iota-, and lambda-carrageenan. Kappa-carrageenan is mostly the alternating polymer of D-galactose-4-sulfate and 3,6-anhydro-D-galactose; iota-carrageenan is similar, except that the 3,6-anhydrogalactose is sulfated at carbon 2. Between kappa-carrageenan and iota-carrageenan there is a continuum of intermediate compositions differing in degree of sulfation at carbon 2. In lambda-carrageenan, the alternating monomeric units are mostly D-galactose-2-sulfate (1,3-linked) and D-galactose-2,6-disulfate (1,4-linked). To determine whether a carrageenan is kappa-, iota- or lambda carrageenan the identity test described in the monograph prepared at the 57^{th} JECFA (2001) can be used.

The term "zinc ionophore" as used herein refers to a substance that reversibly binds zinc ions and that is capable of assisting zinc transport into cells.

The term "terpenoid" as used herein refers to a modified terpene that contain oxygen. Terpenes are a class of natural products consisting of unsaturated hydrocarbons with the formula (C₅H₈)ₙ. Terpenes are further classified by the number of carbons: monoterpenes (C₁₀), sesquiterpenes (C₁₅), diterpenes (C₂₀), etc.

The term "monoterpenoid" as used herein refers to a modified monoterpene that contains oxygen.

The term "sesquiterpenoid" as used herein refers to a modified sesquiterpene that contain oxygen.

In a preferred embodiment, the anti-viral composition of the present invention contains 0.02-2% by weight, more preferably 0.05-1% and most preferably 0.1-0.5% by weight of carrageenan selected from lambda-carrageenan, iota-carrageenan, kappa-carrageenan and combinations thereof.

Preferably, the anti-viral composition contains at least 0.01% by weight, more preferably at least 0.05% by weight and most preferably at least 0.15% by weight of carrageenan selected from lambda-carrageenan, iota-carrageenan and combinations thereof.

In a further preferred embodiment, the anti-viral composition contains at least 0.01% by weight, more preferably at least 0.05% by weight and most preferably at least 0.15% by weight of lambda-carrageenan.

Preferably, at least 30 wt.% of the carrageenan contained in the anti-viral composition is iota-carrageenan and/or lambda-carrageenan. More preferably at least 50 wt.%, most preferably at least 80 wt.% of the carrageenan contained in the anti-viral composition is lambda-carrageenan.

Xylitol is preferably contained in the anti-viral composition in a concentration of 0.3-14% by weight, more preferably in a concentration of 1-12% by weight and most preferably 2-11% by weight.

Preferably, the anionic polysaccharide is contained in the anti-viral composition in a concentration of 0.02-2% by weight, more preferably in a concentration of 0.05-1% by weight.

The anionic polysaccharide is preferably selected from pectin, alginate, gellan gum and combinations thereof. More preferably, the anionic polysaccharide is selected from pectin, alginate and combinations thereof. Even more preferably, the anionic polysaccharide is alginate. The alginate employed preferably is a high G alginate.

The water content of the anti-viral composition of the present invention preferably is in the range of 90-98% by weight, more preferably in the range of 93-97% by weight.

The combination of carrageenan and anionic polysaccharide is preferably present in the anti-viral composition in a concentration of 0.1-5 wt.%, more preferably in a concentration of 0.2 - 2 wt.% and most preferably in a concentration of 0.3-0.8 wt.%.

The anti-viral efficacy of the anti-viral composition of the present invention may be further enhanced by incorporating a zinc ionophore. Accordingly, in a preferred embodiment, the anti-viral composition further comprises 0.001-5.0% by weight, more preferably 0.005-1.0% by weight and most preferably 0.01-0.5% by weight of zinc ionophore.

The zinc ionophore that is employed is preferably selected from hinokitiol, clioquinol, pyrithione, chloroquine, hydroxychloroquine quercetin, epigallocatechol gallate, pyrrolidine dithiocarbamate and combinations thereof. More preferably, the zinc ionophore is selected from hinokitiol, clioquinol, pyrithione and combinations thereof. Most preferably, the zinc ionophore is hinokitiol.

The efficacy of the present anti-viral composition may also be further enhanced by incorporating a a terpenoid selected from monoterpenoid, sesquiterpenoid and combinations thereof.

The anti-viral composition of the present invention preferably contains 0.001-0.5% by weight, more preferably 0.003-0.3% by weight and most preferably 0.005-0.2% by weight of terpenoid selected from monoterpenoid, sesquiterpenoid and combinations thereof.

The terpenoid in the anti-viral composition is preferably selected from 1,8-cineole, menthol, geranial, neral, citronellal, thymol, carvacrol, bisabolol, bisabolol oxide and combinations thereof. More preferably, the terpenoid is selected from 1,8-cineole, menthol, geranial, neral, citronellal and combinations thereof.

According to a particularly preferred embodiment the terpenoid is 1,8-cineole. Preferably, the anti-viral composition contains at least 0.001% by weight, more preferably at least 0.01% by weight of 1,8-cineole. Eucalyptus oil and chamomilla oil may suitably be used as a source of 1,8 cineole.

According to another equally preferred embodiment, the terpenoid is menthol. Preferably, the anti-viral composition contains at least 0.001% by weight, more preferably at least 0.01% by weight of menthol. Mint oils can suitably be used as a source of menthol.

The anti-viral composition of the present invention preferably is an isotonic solution.

Preferably, the anti-viral composition comprises 0.001% to 2.0% by weight, more preferably 0.01-1% by weight of sodium chloride.

The anti-viral composition of the present invention may suitably contain additional components with anti-viral activity such as proton pump inhibitors and antacids (e.g. famotidine, omeprazole, pantoprazole, lansoprozole, tenatoprazole and esomeprazole), and compounds with ACE2 affinity (e.g. remdesivir, lopinavir, sofosbuvir, daclatasvir, ritonavir and naloxegol).

The viscosity of the anti-viral composition preferably is less than 200 mPa.s at 20°C and 16 s⁻¹. More preferably the viscosity is less than 100 mPa.s at 20°C and 16 s⁻¹, most preferably 20-60 mPa.s at 20°C and 16 s⁻¹. The viscosity of the composition may suitably be determined using a Brookfield viscometer model RVDVII+ and using spindle 3.

The anti-viral composition of the present invention preferably is a sterile composition. Sterilization of the composition may be achieved by methods known in the art, such as heat sterilization, high pressure sterilization and filtration sterilization.

According to a further preferred embodiment, the anti-viral composition is buffered to a pH value between 4 and 6.5, more preferably between 5 and 6.

The use of the present anti-viral composition in the prevention or treatment of a viral respiratory infection preferably comprises spraying of the composition into the nose, mouth or throat.

According to a particularly preferred embodiment, the use of the anti-viral composition comprises introducing the composition into the nose.

Typically, the anti-viral composition is administered in a dose of 0.05 to 0.20 ml, more preferably in a dose of 0.075 to 0.15 ml and most preferably of 0.09 to 0.11 ml. The administered dose may be delivered in a single dosage or in multiple dosages, e.g. one dosage into the left nostril and another dosage into the right nostril.

According to a particularly preferred embodiment, the anti-viral composition is used to prevent a viral respiratory infection with one or more viruses selected from coronavirus, rhinovirus, influenza virus, adenovirus and human respiratory syncytial virus. More preferably, the composition is used to prevent a viral respiratory infection with coronavirus. Most preferably, the composition is used to prevent a viral respiratory infection with COVID-19.

Another aspect of the present invention relates to a method of preparing an anti-viral composition as described herein before, said method comprising:
- combining a source of carrageenan, xylitol, a source of anionic polysaccharide and aqueous liquid to produce an aqueous liquid mixture; and
- sterilizing the aqueous liquid mixture;
wherein the source of carrageen contains at least 50% by weight of dry mater of carrageenan selected from lambda-carrageenan, iota-carrageenan, kappa-carrageenan and combinations thereof; wherein the source of anionic polysaccharide contains at least 50% by weight of dry matter of anionic polysaccharide selected from pectin, alginate, gellan gum, xanthan gum and combinations thereof; and wherein the aqueous liquid contains at least 90 wt.% water.

The source of carrageen that is employed preferably contains at least 70% by weight of dry mater, more preferably at least 80% by weight of dry matter of carrageenan selected from lambda-carrageenan, iota-carrageenan, kappa-carrageenan and combinations thereof.

Preferably, the source of carrageenan contains at least 30% by weight of dry matter, more preferably at least 50% by weight of dry matter and most preferably at least 60% by weight of lamba-carrageenan

The source of anionic polysaccharide preferably contains at least 70% by weight of dry matter, more preferably at least 80% by weight of anionic polysaccharide selected from pectin, alginate, gellan gum, xanthan gum and combinations thereof

Preferably, the source of anionic polysaccharide preferably contains at least 70% by weight of dry matter, more preferably at least 80% by weight of anionic polysaccharide selected from pectin, alginate and combinations thereof. Even more preferably, the source of anionic polysaccharide preferably contains at least 70% by weight of dry matter, more preferably at least 80% by weight of alginate. The alginate employed preferably is high G alginate.

In a preferred embodiment, the present method further comprises addition of zinc ionophore prior to the sterilizing of the liquid mixture. The zinc ionophore is preferably selected from hinokitiol, clioquinol, pyrithione, chloroquine, hydroxychloroquine quercetin, epigallocatechol gallate, pyrrolidine dithiocarbamate and combinations thereof. More preferably, the zinc ionophore is selected from hinokitiol, clioquinol, pyrithione and combinations thereof. Most preferably, the zinc ionophore is hinokitiol.

According to yet another preferred embodiment, the method further comprises addition of a source of terpenoid, wherein the source of terpenoid contains at least 40% by weight of dry matter of monoterpenoid, sesquiterpenoid and combinations thereof.

The source of terpenoid that is employed in the preparation method preferably is selected from eucalyptus oil, mint oil, chamomilla oil, melissa oil, thyme oil, oregano oil, lemongrass oil and combinations thereof.

The source of terpenoid preferably contains at least 50% by weight of dry matter, more preferably at least 70% by weight of dry matter and most preferably 80-100% by weight of dry matter of terpenoid selected from monoterpenoid, sesquiterpenoid and combinations thereof.

According to a particularly preferred embodiment, the source of terpenoid is eucalyptus oil. Preferably, the eucalyptus oil contains at least 15 wt.%, more preferably at least 30 wt.% and most preferably 50-90 wt.% of1,8-cineole.

According to another particularly preferred embodiment, the source of terpenoid is mint oil. Examples of mint oils that may be employed include peppermint oil, spearmint oil, and combinations thereof. Preferably, the mint oil contains at least 30 wt.%, more preferably at least 40 wt.% and most preferably 50-70 wt.% menthol.

Yet another aspect of the invention relates to a metered-dose pump spray device comprising
- a container holding the anti-viral composition as defined herein before,
- a nozzle, and
- a manual pump connecting the nozzle and the container, said pump being configured to deliver one spray-unit upon actuation by manual force.

The one spray-unit that is delivered by the device upon actuation preferably has a volume between 1 and 30 ml, more preferably between 5 and 20 ml.

The invention is further illustrated by the following non-limiting examples.

### EXAMPLES

### Example 1

Aqueous liquid formulations were prepared on the basis of the recipes shown in Table 1.

**Table 1**

| | **Wt.%** | | | | | |
|---|---|---|---|---|---|---|
| | **1** | **2** | **3** | **4** | **5** | **6** |
| Lambda carrageenan ¹ | 0.200 | 0.200 | | 0.200 | 0.200 | 0.200 |
| Xylitol | 5 | | 5 | 5 | 5 | 5 |
| Alginate high G ² | | 0.050 | 0.050 | 0.050 | 0.050 | 0.050 |
| Hinokitiol | | | | | 0.1 | |
| Eucalyptus globulus oil ³ | | | | | | 0.050 |
| Ethanol | 0.500 | 0.500 | 0.500 | 0.500 | 0.500 | 0.500 |
| Sodium octanoate | 0.200 | 0.200 | 0.200 | 0.200 | 0.200 | 0.200 |
| Adjust pH to 6.0 with HCI/NaOH 2M | | | | | | |
| Aqua purificata | Ad 100 | Ad 100 | Ad 100 | Ad 100 | Ad 100 | Ad 100 |

| | | | | | | |
|---|---|---|---|---|---|---|
| ¹ Viscarin PH 109 NF (ex FMC biopolymer) ² Alginic acid sodium salt (ex Carl Roth) ³ Contains 69 wt.% 1,8-cineol | | | | | | |

The anti-viral activity of these formulations against coronavirus SARS-CoV-2 is tested in three different cell-based assays (all tests carried out at 37°C on Vero E6 cells).

In a first assay the direct antiviral activity is determined. Virus suspensions are pre-incubated with the antiviral formulations for 10 minutes, then added to the cell culture. After 3 days of incubation, the resulting viral load is assayed by titration.

In a second cell-based assay the effect of exposure of cells to the formulations prior to exposure to corona virus is determined. Cells are incubated with formulations for 2 hours, then coronavirus is added. After three days of incubation the viral load is assayed by titration.

The third cell-based assay is identical to the second cell-based assay, except that formulation, virus and human cell-line are combined in one step.

An overall score for the anti-viral effect is calculated for each formulation by multiplying the scores obtained from the three above mentioned assays. The results show that the anti-viral activity of formulation 4 was substantially higher than the anti-viral activity of each of the formulations 1, 2 and 3.

Furthermore, results show that the antiviral activity of both formulation 5 and formulation 6 was higher than the antiviral activity of formulation 4.

### Example 2

In order to simulate the effective contact time of formulations 1 and 4 of Example 1, the following experiment is conducted.

Sheets of paper are drenched in simulated nasal slime, or alternatively in demi-water. Formulations 1 and 4 are dyed with a water-soluble dye and sprayed onto both types of pretreated papers. After 1 minute the paper sheets are flushed 100 times with 0.1 ml demineralized water or, alternatively, the sheets are immersed in 500 mL demineralized water for 1 hour. After these treatments, the colour intensity of the paper sheets is measured.

The results show that the color intensity of the paper sheets that had been sprayed with formulation 4 is much higher than the color intensity of the paper sheets that had been sprayed with formulation 1.

### Example 3

An aqueous liquid anti-viral formulation was prepared on the basis of the recipe shown in Table 2, using the same ingredients as in Example 1.

**Table 2**

| | **Wt.%** |
|---|---|
| Lambda carrageenan | 0.10 |
| Alginate high G | 0.20 |
| Xylitol | 5.0 |
| Eucalyptus globulus oil | 0.10 |
| Ethanol | 0.5 |
| Sodium octanoate | 0.20 |
| Tween 80 | 0.20 |
| Benzalkonium chloride (50% aq. solution) | 0.01 |
| Adjust pH to 6.0 with HCI/NaOH 2M | |
| Aqua purificata | Ad 100 |

The anti-viral formulation was administered to a human subject by spraying 0.1 mL of the formulation into each nostril. This showed that the formulation was well tolerated.

## Claims

1. An aqueous liquid composition for use in the prevention or treatment of a viral respiratory infection, said use comprising introducing the liquid aqueous composition into nose, mouth or throat of a human subject, said aqueous composition comprising:
• 0.01-5% by weight of carrageenan selected from lambda-carrageenan, iota-carrageenan, kappa-carrageenan and combinations thereof;
• 0.1-15% by weight of xylitol; and
• 0.01-5% by weight of anionic polysaccharide selected from pectin, alginate, gellan gum, xanthan gum and combinations thereof;
• 80-99% by weight water.

2. Composition for use in the prevention or treatment of a viral respiratory infection according to claim 1, wherein the carrageenan is lambda-carrageenan.

3. Composition for use in the prevention or treatment of a viral respiratory infection according to claim 1 or 2, wherein the anionic polysaccharide is selected from pectin, alginate and combinations thereof.

4. Composition for use in the prevention or treatment of a viral respiratory infection according to any one of the preceding claims, wherein the composition further comprises 0.001-5wt.% of zinc ionophore.

5. Composition for use in the prevention or treatment of a viral respiratory infection according to claim 4, wherein the zinc ionophore is selected from hinokitiol, pyrithione, clioquinol, chloroquine, hydroxychloroquine quercetin, epigallocatechol gallate, pyrrolidine dithiocarbamate, and combinations thereof.

6. Composition for use in the prevention or treatment of a viral respiratory infection according to any one of the preceding claims, wherein the composition further comprises 0.001-1% by weight of terpenoid selected from monoterpenoid, sesquiterpenoid and combinations thereof.

7. Composition for use in the prevention or treatment of a viral respiratory infection according to claim 6, wherein the terpenoid is selected from 1,8-cineole, menthol, geranial, neral, citronellal, thymol, carvacrol, bisabolol, bisabolol oxide and combinations thereof.

8. Composition for use in the prevention or treatment of a viral respiratory infection according to claim 7, wherein the terpenoid is 1,8-cineole.

9. Composition for use in the prevention or treatment of a viral respiratory infection according to claim 7, wherein the terpenoid is menthol.

10. Composition for use in the prevention or treatment of a viral respiratory infection according to any one of the preceding claims, wherein the use comprises introducing the composition into the nose.

11. Composition for use in the prevention or treatment of a viral respiratory infection according to any one of the preceding claims, wherein the composition is used to prevent infection with one or more viruses selected from coronavirus, rhinovirus, influenza virus, adenovirus and human respiratory syncytial virus.

12. Composition for use in the prevention or treatment of a viral respiratory infection according to claim 11, wherein the composition is used to prevent infection with coronavirus.

13. A method of preparing a liquid aqueous composition as defined in any one of claims 1-9, said method comprising:
• combining a source of carrageenan, xylitol, a source of anionic polysaccharide and aqueous liquid to produce an aqueous liquid mixture; and
• sterilizing the aqueous liquid mixture;
wherein the source of carrageen contains at least 50% by weight of dry mater of carrageenan selected from lambda-carrageenan, iota-carrageenan, kappa-carrageenan and combinations thereof; wherein the source of anionic polysaccharide contains at least 50% by weight of dry matter of anionic polysaccharide selected from pectin, alginate, gellan gum, xanthan gum and combinations thereof; and wherein the aqueous liquid contains at least 90 wt.% water.

14. A metered-dose pump spray device comprising
• a container holding the aqueous composition as defined in any one of claims 1-11,
• a nozzle, and
• a manual pump connecting the nozzle and the container, said pump being configured to deliver one spray-unit upon actuation by manual force.

15. Metered-dose pump spray device according to claim 14, wherein the one spray-unit has a volume between 1 and 30 ml.
